# EUROPÄISCHE PATENTSCHRIFT

(11) **EP 1 532 986 B1**
(45) Veröffentlichungstag und Bekanntmachung des Hinweises auf die Patenterteilung: **16.12.2009**
(21) Anmeldenummer: 04024727.2
(22) Anmeldetag: 16.10.2004
(51) Int. Cl.: A61K 47/36, A61K 31/4174, A61P 11/02, A61K 45/06, A61K 31/728

(54) **Pharmazeutische Zusammensetzung zur Behandlung von Rhinitiden**
Pharmaceutical composition for treatment of rhinitis
Composition pharmaceutique pour le traitement de la rhinite

(30) Priorität: 13.11.2003 DE 10353690; 02.12.2003 DE 10356248
(43) Veröffentlichungstag der Anmeldung: 25.05.2005
(73) Patentinhaber: Maria Clementine Martin Klosterfrau Vertriebsgesellschaft mbH, 50670 Köln (DE)
(72) Erfinder: Greve, Harald, Dr., 40545 Düsseldorf (DE); Greve, Rainer, Dr., 23795 Bad Segeberg (DE)
(74) Vertreter: Gesthuysen, von Rohr & Eggert

(56) Entgegenhaltungen:
- EP-A- 0 773 022
- WO-A-02/051380
- WO-A-02/064113
- WO-A-03/049747
- WO-A-2004/000272
- GB-A- 1 087 842
- PATENT ABSTRACTS OF JAPAN Bd. 1995, Nr. 04, 31. Mai 1995 (1995-05-31) & JP 07 017870 A (TOSHIKO YAMAMOTO), 20. Januar 1995 (1995-01-20) & DATABASE WPI Week 199513 Derwent Publications Ltd., London, GB; AN 1995-093767 "Troches having antiinflammatory and stable moisturising activities - comprises antiinflammatory agent e.g. chloro-hexadiene chloride, and hyaluronic acid" & JP 07 017870 A (YAMAMOTO T) 20. Januar 1995 (1995-01-20)

## Beschreibung

Die vorliegende Erfindung betrifft eine pharmazeutische Zusammensetzung auf Basis eines Sympathomimeticums mit vasokonstriktorischer bzw. schleimhautabschwellender Wirkung in Kombination mit Hyaluronsäure oder deren Salzen und mit Pantothenol oder dessen Estern und/oder Pantothensäure oder deren physiologisch unbedenklichen Salzen, wobei sich die Zusammensetzung zur prophylaktischen und/oder kurativen topischen Behandlung von Rhinitiden eignet, sowie deren bestimmungsgemäße Verwendung zur prophylaktischen und/oder kurativen topischen Behandlung von Rhinitiden aller Art, insbesondere akuter wie chronischer Rhinitiden.

Für die Behandlung von Rhinitiden steht eine Vielzahl von Sympathomimetica mit vasokonstriktorischen bzw. schleimhautabschwellenden Eigenschaften zur Verfügung, welche bei topischer bzw. lokaler Anwendung in der Nase zur Nasenschleimhautabschwellung führen. Eine wiederholte Anwendung dieser Substanzen führt jedoch häufig zu einer Austrocknung der Nasenschleimhäute, verbunden mit entzündlichen Irritationen. Diese Nebenwirkungen führen nicht selten zu einer erhöhten Infektionsgefahr, da die Schleimhäute im ausgetrockneten und entzündeten Zustand nicht mehr ihre Schutz- und Filterfunktionen im vollen Umfang aufrechterhalten können, so daß Krankheitserreger ungehinderter in die Atemwege gelangen können.

Um diesen allgemein bekannten Nebenwirkungen von Sympathomimetica entgegenzuwirken, wird in den auf die Anmelderin selbst zurückgehenden Patentanmeldungen DE 195 41 919 A1 und DE 195 49 421 A1 bzw. EP 0 773 022 A2 vorgeschlagen, pharmazeutischen Zubereitungen zur Behandlung von Rhinitiden auf Basis vasokonstriktorisch bzw. schleimhautabschwellend wirkender Sympathomimetica wirksame Mengen Pantothenol oder Pantothensäure zuzusetzen. Denn Pantothenol oder Pantothensäure in Kombination mit einem zur topischen Anwendung geeigneten Sympathomimeticum wirkt einer Austrocknung und somit einer entzündlichen Irritation der Nasenschleimhäute entgegen.

Die WO 2004/000272 A1 welche Stand der Technik nach Artikel 54(3) EPÜ bildet, beschreibt nasale Zusammensetzungen mit einem Mucopolysaccharid und Propylenglykol. In Ausführungsbeispiel 3 auf den Seiten 7/8 von D1 ist eine Nasensprayzusammensetzung beschrieben, welche neben 0,1 Gew.-% Xylometazolinhydrochlorid und 2,0 Gew.-% Propylenglykol auch 0,1 Gel.-% Natriumhyaluronat enthält.

Das JAPIO-Abstract zu der JP 07-017870 A beschreibt eine antiinflammatorisch wirkende Zusammensetzung mit befeuchtender Wirkung in bezug auf die Mundhöhle oder den Rachenbereich, wobei die dort beschriebene Zusammensetzung einen antiinflammatorisch wirkenden Wirkstoff sowie Hyaluronsäure umfaßt. Es wird keine nasale Applikation der dort beschriebenen Zusammensetzung in Betracht gezogen.

Die WO 02/051380 A1 betrifft eine pharmazeutische Zusammensetzung aus mindestens einem wasserlöslichen oder schwer wasserlöslichen Wirkstoff und Hyaluronsäure bzw. deren Derivaten sowie gegebenenfalls einem Lösungsvermittler und/oder Hilfsstoff.

Die GB 1 087 842 A offenbart eine topische Zusammensetzung für die nasale Applikation, welche eine antienzymatische organische Verbindung mit einem Molekulargewicht von 2.000 bis 50.000 in Form eines speziellen Kondensationsproduktes enthält, welches aromatische Kerne aufweist.

Weiterhin beschreibt die WO 03/049747 A1 eine pharmazeutische Zusammensetzung zur ophthalmologischen oder rhinologischen Anwendung, welche Pantothenol und/oder Pantothensäure zusammen mit Hyaluronsäure und/oder Hyaluronat enthält.

Der vorliegenden Erfindung liegt nun die Aufgabe zugrunde, eine pharmazeutische Zusammensetzung bereitzustellen, welche sich zur topischen Behandlung von Rhinitiden eignet und insbesondere die zuvor geschilderten Nachteile des Standes der Technik zumindest weitgehend vermeidet oder wenigstens abschwächt.

Eine weitere Aufgabe der vorliegenden Erfindung liegt darin, eine pharmazeutische Zusammensetzung auf Basis von Sympathomimetica mit vasokonstriktorischen bzw. schleimhautabschwellenden Eigenschaften bereitzustellen, welche ein Austrocknen und entzündliche Irritationen der Nasenschleimhäute vermeidet. Insbesondere soll dabei eine Alternative zu den aus dem Stand der Technik bekannten, auf die Anmelderin selbst zurückgehenden Zusammensetzungen gemäß DE 195 41 919 A1 und DE 195 49 421 A1 bereitgestellt werden bzw. die dort beschriebenen Zusammensetzungen weiterentwickelt werden.

Die Anmelderin hat nun überraschenderweise herausgefunden, daß das zuvor geschilderte Problem dadurch gelöst werden kann, daß man eine pharmazeutische Zubereitung auf Basis eines zur topischen Anwendung geeigneten Sympathomimeticums mit vasokonstrikorischer bzw. schleimhautabschwellender Wirkung oder dessen physiologisch unbedenklichen Salzen zusammen mit Hyaluronsäure oder deren physiologisch unbedenklichen Salzen und mit Pantothenol oder dessen Estern und/oder Pantothensäure oder deren physiologisch unbedenklichen Salzen formuliert.

Zur Lösung des zuvor geschilderten Problems schlägt die vorliegenden Erfindung - gemäß einem ersten Aspekt der vorliegenden Erfindung - eine zur prophylaktischen und/oder kurativen topischen Behandlung von Rhinitiden bestimmte pharmazeutische Zusammensetzung nach Patentanspruch 1 vor; weitere, vorteilhafte Ausgestaltungen sind Gegenstand der diesbezüglichen Unteransprüche (Patentansprüche 2 bis 11).

Weiterer Gegenstand der vorliegenden Erfindung ist - gemäß einem weiteren, zweiten Aspekt der vorliegenden Erfindung - die Verwendung der erfindungsgemäßen pharmazeutischen Zusammensetzung, wie sie in Patentanspruch 12 definiert ist; weitere, vorteilhafte Ausgestaltungen dieses Erfindungsaspektes sind Gegenstand der diesbezüglichen Verwendungsunteransprüche (Patentansprüche 13 bis 15).

Gegenstand der vorliegenden Erfindung gemäß dem ersten Erfindungsaspekt ist somit eine pharmazeutische Zusammensetzung, welche sich zur prophylaktischen und/oder kurativen topischen Behandlung von Rhinitiden eignet, wobei die pharmazeutische Zusammensetzung in Kombination und in jeweils pharmazeutisch wirksamen Mengen
a) 0,001 bis 1 Gew.-% mindestens eines zur topischen Anwendung geeigneten Sympathomimeticums mit vasokonstriktorischer und/oder schleimhautabschwellender Wirkung oder dessen physiologisch unbedenkliche Salze;
b) 0,01 bis 5 Gew.-% Hyaluronsäure oder deren physiologisch unbedenkliche Salze; und
c) 0,01 bis 15 Gew.-% Pantothenol oder dessen Ester und/oder Pantothensäure oder deren physiologisch unbedenkliche Salze,
   enthält.

Überraschenderweise wirkt die Hyaluronsäure oder deren physiologisch verträgliche bzw. physiologisch unbedenkliche Salze zusammen mit Pantothenol bzw. dessen Estern und/oder Pantothensäure bzw. deren physiologisch unbedenklichen Salzen einer durch die Anwendung des Sympathomimeticums bedingten Austrocknung der Nasenschleimhäute entgegen und verhindert auf diese Weise entzündliche Irritationen der Nasenschleimhäute.

Erfindungsgemäß besonders bevorzugt wird die Hyaluronsäure in Form ihrer physiologisch verträglichen Salze, bevorzugt in Form ihres Natriumsalzes eingesetzt. Hyaluronsäure (Hyaluronan) ist ein saures Glykosaminoglykan (Mukopolysaccharid) biologischen Ursprungs, welches erstmalig aus dem Glaskörper von Rinderaugen isoliert worden ist und welches auch in der Synovialflüssigkeit der Gelenke sowie in der Haut vorkommt, wo es mehr als 50 % der Hautgrundsubstanz bildet. Hyaluronsäure und ihre Derivate, insbesondere ihre Salze, zeichnen sich durch eine hohe Wasserbindungsfähigkeit aus; Hyaluronsäure ist eine hochviskose, wäßrige Lösungen bildende, hochmolekulare Verbindung, für die je nach Herkunft, Aufarbeitungs- und Bestimmungsmethoden Molmassen zwischen 50.000 und mehreren Millionen angegeben werden. Für weitere Einzelheiten kann beispielsweise verwiesen werden auf Römpp Chemielexikon, 10. Auflage, Band 3, Seite 1820, Stichwort: "Hyaluronsäure", Georg Thieme Verlag Stuttgart/New York, 1997.

Es ist vollkommend überraschend, daß die Verwendung von Hyaluronsäure oder deren Salzen, in Kombination mit einem zur topischen Anwendung geeigneten Sympathomimeticum mit vasokonstriktorischer bzw. schleimhautabschwellender Wirkung oder dessen physiologisch unbedenklichen Salzen sowie mit Pantothenol bzw. dessen Estern und/oder Pantothensäure bzw. deren physiologisch verträglichen Salzen zur Behandlung von Rhinitiden sehr viel besser geeignet ist als bekannte Monopräparate, welche nur das Sympathomimeticum enthalten, und daß durch einen synergistischen Effekt des Zusammenwirkens der Hyaluronsäure bzw. deren Salzen sowie Pantothenol bzw. Pantothensäure mit dem Sympathomimeticum ein Austrocknen und entzündliche Irritationen der Nasenschleimhäute bei der erfindungsgemäßen topischen bzw. lokalen Anwendung der pharmazeutischen Zusammensetzung vermieden werden. Unerwarteterweise wurde in Studien der Anmelderin aufgrund des synergistischen Zusammenwirkens des Sympathomimeticums mit der Hyaluronsäure sowie Pantothenol bzw. Pantothensäure ein deutlich beschleunigter Heilungsverlauf der Rhinitiden, insbesondere akuter Rhinitiden, unter topischer Anwendung der erfindungsgemäßen pharmazeutischen Zusammensetzung beobachtet.

Die Menge an Hyaluronsäure oder deren physiologisch unbedenklichen Salzen in der erfindungsgemäßen pharmazeutischen Zusammensetzung kann in weiten Bereichen variieren. Im allgemeinen ist die Hyaluronsäure oder deren physiologisch unbedenkliche Salze in Mengen von 0,01 bis 5 Gew.-%, insbesondere 0,05 bis 1 Gew.-%, vorzugsweise 0,05 bis 0,25 Gew.-%, bezogen auf die pharmazeutische Zusammensetzung, enthalten. Bei signifikanter Unterschreitung der Untergrenze von 0,01 Gew.-% wird im allgemeinen eine zu geringe Wirkung erreicht, wohingegen im Fall zu großer Konzentrationen deutlich oberhalb von 5 Gew.-% im allgemeinen eine zu starke Verdickung der erfindungsgemäßen pharmazeutischen Zusammensetzung beobachtet wird. Dennoch kann es gegebenenfalls erforderlich sein, von den genannten Mengen abzuweichen, sofern die therapeutischen Gegebenheiten dies erfordern.

Als Sympathomimetica mit vasokonstriktorischer bzw. schleimhautabschwellender Wirkung können erfindungsgemäß alle aus dem Stand der Technik für diese Zwecke bekannten Sympathomimetica in Betracht kommen, insbesondere zur topischen Anwendung geeignete α-Sympathomimetica, vorzugsweise α-Sympathomimetica mit 2-Imidazolinstruktur, oder deren physiologisch unbedenkliche Salze.

Sympathomimetisch wirksame Imidazolinderivate verursachen bekanntermaßen eine Kontraktion der Gefäße, insbesondere in der Schleimhaut, und werden daher lokal zur Abschwellung der Schleimhaut im Nasenraum gegeben. Die Sekretionshemmung und Abschwellung der Schleimhäute führen zu einer Linderung der Rhinitiden. Solche α-Sympathomimetica auf Basis von Imidazolinderivaten, wie z. B. Oxymetazolin, Xylometazolin, Tramazolin, Tetryzolin und Naphazolin, stimulieren direkt die α-adrenergen Rezeptoren des sympathischen Nervensystems, haben jedoch wenig oder keine Wirkung auf βadrenerge Rezeptoren. Die intranasale Applikation solcher (α-Sympathomimetica führt im allgemeinen zur Konstriktion dilatierter Arteriolen und damit zur Normalisierung der vermehrten Schleimhautdurchblutung, zur Reduktion der Ödembildung und zur Verbesserung der nasalen Ventilation. Durch die Belüftung der Nebenhöhlen und der Tube verringert sich die Gefahr von Komplikationen, z. B. infolge eines Sekretstaus. Nach intranasaler Applikation von α-Sympathomimetica tritt eine lokale Vasokonstriktion gewöhnlich innerhalb von kurzen Zeiträumen, beispielsweise innerhalb von 5 bis 10 Minuten, auf und persistiert im allgemeinen für mehrere Stunden.

Als Folge eines sogenannten "Rebound-Effektes" kann es nach wiederholter Anwendung von Sympathomimetica zu einer medikamentös bedingten Rhinitis mit Austrocknungen und entzündlichen Irritationen an der Nasenschleimhaut kommen, so daß die vielfältigen therapeutischen Anwendungsmöglichkeiten der Sympathomimetica weitgehend eingeschränkt sind. Es wurde nun aber überraschend gefunden, daß bei lokaler Anwendung von Hyaluronsäure oder deren Salzen zusammen mit Pantothenol bzw. Pantothensäure, in Kombination mit Sympathomimetica auf der Nasenschleimhaut bereits nach kurzen Behandlungszeiten ein manifester Heilungserfolg verzeichnet wird und entzündliche Irritationen und Austrocknungen der Nasenschleimhäute vermieden werden.

Erfindungsgemäß bevorzugte Beispiele für zur topischen Anwendung geeignete α-Sympathomimetica mit 2-Imidazolinstruktur oder deren physiologisch unbedenkliche Salze sind Oxymetazolin, Xylometazolin, Tramazolin, Tetryzolin und Naphazolin sowie deren physiologisch unbedenkliche Salze, insbesondere deren Hydrochloride.

Erfindungsgemäß besonders bevorzugt sind Oxymetazolin und Xylometazolin sowie deren physiologisch unbedenkliche Salze, insbesondere deren Hydrochloride.

Als erfindungsgemäß verwendbare Sympathomimetica eignen sich aber auch Ephedrin und Ephedrinderivate, wie Pseudoephedrin, Norephedrin, Norpseudoephedrin, N-Methylephedrin und N-Methylpseudoephedrin. Erfindungsgemäß ganz besonders bevorzugt ist (-)-Ephedrin bzw. (1R,2S)-2-Methylamino-1-phenyl-1-propanol. Ephedrin und Ephedrinderivate sind Alkaloide aus Ephedra-Arten. Sie sind indirekt wirkende Sympathomimetica mit vasokonstriktorischer Wirkung. Besonders wirksam ist das natürlich vorkommende (-)-Ephedrin bzw. (1R,2S)-2-Methylamino-1-phenyl-1-propanol. Die hierzu enantiomere Verbindung (1S,2R)-Ephedrin zeigt nur etwa ein Drittel der pharmakologischen Wirkung der natürlichen Form. Die beiden Diastereomeren von Ephedrin und seinem synthetischen Enantiomer werden Pseudoephedrine genannt. Für weitere Einzelheiten zu Ephedrin und Ephedrinderivaten kann verwiesen werden auf Römpp Chemielexikon, 10. Auflage, Band 2, Seiten 1181/1182, Stichwort: "Ephedrin", Georg Thieme Verlag Stuttgart/New York, 1997 und die dort referierte Literatur sowie auf ROCHE-Lexikon Medizin, 3. Auflage, Seite 478, Stichwort: "Ephedrin", Verlag Urban & Schwarzenberg, wobei der Inhalt der vorgenannten Literatur hiermit in vollem Umfang durch Bezugnahme eingeschlossen ist.

Die Menge an Sympathomimeticum in der erfindungsgemäßen pharmazeutischen Zusammensetzung kann in weiten Bereichen variieren. Im allgemeinen ist das Sympathomimeticum in Mengen von 0,001 bis 1 Gew.-%, insbesondere 0,01 bis 0,1 Gew.-%, vorzugsweise 0,01 bis 0,05 Gew.-%, bezogen auf die pharmazeutische Zusammensetzung, enthalten. Bei signifikanter Unterschreitung der Untergrenze von 0,001 Gew.-% wird im allgemeinen eine zu geringe Wirkung erreicht, wohingegen zu große Konzentrationen deutlich oberhalb von 1 Gew.-% im allgemeinen zu keiner nennenswerten Steigerung des therapeutischen Effekts führen. Dennoch kann es gegebenenfalls erforderlich sein, von den genannten Mengen abzuweichen, sofern die therapeutischen Gegebenheiten dies erfordern.

Wie zuvor ausgeführt, sind der pharmazeutischen Zusammensetzung außerdem noch als weitere Komponente - in Kombination mit dem Sympathomimeticum und der Hyaluronsäure - zusätzlich auch Pantothenol oder dessen physiologisch unbedenkliche Derivate, nämlich Ester, und/oder Pantothensäure oder deren physiologisch unbedenkliche Salze zugesetzt sind. Besonders bevorzugt sind D(+)-Pantothenol (Dexpanthenol) oder dessen physiologisch unbedenkliche Derivate, nämlich dessen Ester.

Es war nicht zu erwarten, daß das Pantothenol bzw. seine Ester und/oder die Pantothensäure bzw. deren physiologisch unbedenkliche Salze in synergistischer Kombination mit dem Sympathomimeticum und der Hyaluronsäure oder deren Salzen, zu einer besonders verbesserten pharmazeutischen Zusammensetzung für die Behandlung von Rhinitiden führt, weil eine derartige Zusammensetzung neben einer vasokonstriktorischen bzw. schleimhautabschwellenden Wirkung jeglicher Austrocknung mit einhergehenden entzündlichen Irritationen der Nasenschleimhäute besonders gut entgegenwirkt bzw. diese vermeidet.

Die Menge an Pantothenol bzw. seinen Estern und/oder Pantothensäure bzw. deren physiologisch unbedenklichen Salzen in der erfindungsgemäßen pharmazeutischen Zusammensetzung kann in weiten Bereichen variieren. Im allgemeinen enthält die erfindungsgemäße pharmazeutische Zusammensetzung Pantothenol oder dessen Ester und/oder Pantothensäure oder deren physiologisch unbedenkliche Salze in Mengen von insgesamt 0,01 bis 15 Gew.-%, insbesondere 0,1 bis 10 Gew.-%, vorzugsweise 0,2 bis 5 Gew.-%, bezogen auf die pharmazeutische Zusammensetzung. Dennoch kann es gegebenenfalls erforderlich sein, von den genannten Mengen abzuweichen, sofern die therapeutischen Gegebenheiten dies erfordern.

Die Mengenverhältnisse der einzelnen Bestandteile a) bis c) in der erfindungsgemäßen pharmazeutischen Zusammensetzung können in weiten Bereichen variieren: Insbesondere enthält die pharmazeutische Zusammensetzung nach der vorliegenden Erfindung die Komponenten a) und b) in gewichtsbezogenen Mengenverhältnissen von a) : b) im Bereich von 100 : 1 bis 1 : 5.000 und/oder die Komponenten a) und c) in gewichtsbezogenen Mengenverhältnissen von a) : c) im Bereich von 100 : 1 bis 1 : 15.000 und/oder die Komponenten b) und c) in gewichtsbezogenen Mengenverhältnissen von b) : c) im Bereich von 500 : 1 bis 1 : 1.500.

Eine erfindungsgemäß bevorzugte pharmazeutische Zusammensetzung zur prophylaktischen und/oder kurativen topischen Behandlung von Rhinitiden enthält - in Kombination und jeweils bezogen auf die pharmazeutische Zusammensetzung -
a) 0,01 bis 0,1 Gew.-%, vorzugsweise 0,01 bis 0,05 Gew.-%, mindestens eines zur topischen Anwendung geeigneten Sympathomimeticums mit vasokonstriktorischer und/oder schleimhautabschwellender Wirkung oder dessen physiologisch unbedenkliche Salze;
b) 0,05 bis 1 Gew.-%, vorzugsweise 0,05 bis 0,25 Gew.-%, Hyaluronsäure oder deren physiologisch unbedenkliche Salze; und
c) 0,1 bis 10 Gew.-%, vorzugsweise 0,2 bis 5 Gew.-%, Pantothenol oder dessen Ester und/oder Pantothensäure oder deren physiologisch unbedenkliche Salze.

Eine erfindungsgemäß besonders bevorzugte pharmazeutische Zusammensetzung, welche sich insbesondere zur prophylaktischen und/oder kurativen topischen Behandlung von Rhinitiden eignet, enthält - in Kombination und jeweils bezogen auf die pharmazeutische Zusammensetzung -
a) 0,001 bis 1 Gew.-%, insbesondere 0,01 bis 0,1 Gew.-%, vorzugsweise 0,01 bis 0,05 Gew.-%, Oxymetazolin und/oder Xylometazolin, vorzugsweise in Form ihrer physiologisch unbedenklichen Salze, insbesondere in Hydrochloridform;
b) 0,01 bis 5 Gew.-%, insbesondere 0,05 bis 1 Gew.-%, vorzugsweise 0,05 bis 0,25 Gew.-%, Hyaluronsäure, vorzugsweise in Form ihrer physiologisch unbedenklichen Salze, insbesondere als Natriumsalz; und
c) 0,01 bis 15 Gew.-%, insbesondere 0,1 bis 10 Gew.-%, vorzugsweise 0,2 bis 5 Gew.-%, Pantothenol oder dessen Ester und/oder Pantothensäure oder deren physiologisch unbedenkliche Salze.

Die erfindungsgemäße pharmazeutische Zusammensetzung kann eine flüssige oder dickflüssige bis halbfeste Konsistenz aufweisen. Beispielsweise kann die erfindungsgemäße pharmazeutische Zusammensetzung z. B. als Salbe, Creme oder Gel zum Einbringen in die Nase oder als Lösung oder Dispersion zum Tropfen oder Sprühen in die Nase vorliegen.

Als Träger für flüssige Darreichungsformen eignen sich insbesondere wäßrige Systeme mit oder ohne Zusatz von Glycerin, Sorbit oder anderen Polyolen. Als Trägerstoffe für dickflüssige oder halbfeste pharmazeutische Zubereitungen, wie zum Beispiel Salben, Cremes oder Gelen, eignen sich zum Beispiel Paraffinkohlenwasserstoffe, Vaseline, Wollwachsprodukte und andere pharmazeutisch verwendbare, viskositätserhöhende Grundstoffe, bei hydrophilen Gelen zum Beispiel Wasser, Glycerin oder Sorbit, die mit geeigneten Quellstoffen, wie z. B. Polyacrylsäure, Cellulosederivate, Stärke oder Traganth, geliert werden.

Die erfindungsgemäße pharmazeutische Zusammensetzung kann neben den Wirk- und Trägersubstanzen und gegebenenfalls vorhandenen Emulgatoren noch andere unbedenkliche und in bezug auf die Wirkstoffe kompatible pharmazeutische Hilfs- und/oder Zusatzstoffe enthalten, so z. B. Füll-, Streck-, Binde-, Netz-, Stabilisierungs-, Färbe-, Puffer- und Riechstoffe. Weiterhin können mikrobiologisch aktive chemische Verbindungen, wie z. B. Konservierungsstoffe oder Antiseptika zur Verbesserung der mikrobiellen Stabilität, in pharmazeutisch üblichen Konzentrationen in der erfindungsgemäßen pharmazeutischen Zusammensetzung enthalten sein. Darüber hinaus kann die erfindungsgemäße pharmazeutische Zusammensetzung auch noch eine oder mehrere andere pharmakologisch wirksame Substanzen enthalten.

Die Herstellung der erfindungsgemäßen pharmazeutischen Zusammensetzung erfolgt in an sich bekannter Weise. Dies geschieht beispielsweise durch Mischen bzw. Lösen der Wirkstoffe in pharmakologisch wirksamen Konzentrationen, der Hilfs- und/oder Zusatzstoffe sowie der gegebenenfalls weiteren pharmakologisch wirksamen Substanzen in dem vorgesehenen Trägermedium.

Zum Nachweis der Wirkung, insbesondere der synergistischen Wirkung, der erfindungsgemäßen pharmazeutischen Zusammensetzung wurde in Versuchen jeweils eine Nasenseite mit einem Sympathomimeticum allein und die andere Nasenseite mit einer erfindungsgemäßen pharmazeutischen Zusammensetzung behandelt. In diesen Versuchen ergab die laufende Rhinoskopie bei den mit der erfindungsgemäßen pharmazeutischen Zusammensetzung behandelten Nasenseite eine deutliche Verbesserung gegenüber der mit dem Sympathomimeticum allein behandelten Nasenseite. Darüber hinaus zeigte sich, daß die Hyaluronsäure bzw. deren Salze die Reizwirkung des Sympathomimeticums und ein Austrocknen der Schleimhäute und folglich infektiöse Irritationen linderte bzw. gänzlich verhinderte, wobei dieser Effekt durch Pantothenol bzw. Pantothensäure noch weiter gesteigert wurde.

Weiterer Gegenstand der vorliegenden Erfindung ist die Verwendung der pharmazeutischen Zusammensetzung nach der vorliegenden Erfindung zur prophylaktischen und/oder kurativen topischen Behandlung von Rhinitiden bzw. zur Herstellung eines Arzneimittels zur prophylaktischen und/oder kurativen topischen Behandlung von Rhinitiden.

Die erfindungsgemäße pharmazeutische Zusammensetzung eignet sich zur Behandlung von Rhinitiden aller Art, und zwar akuter wie chronischer Rhinitiden. Beispielhaft seien in diesem Zusammenhang Rhinitis acuta, Rhinitis allergica, Rhinitis atrophicans, Rhinitis hyperplastica oder hypertrophicans, Rhinitis mutilans, Rhinitis nervosa oder vasomotorica, Rhinitis pseudomembranacea und Rhinitis sicca genannt. Besonders eignet sich die erfindungsgemäße pharmazeutische Zusammensetzung zur Behandlung akuter Rhinitiden. Aber auch chronische Rhinitiden lassen sich mit der erfindungsgemäßen pharmazeutischen Zusammensetzung gut therapieren, insbesondere aufgrund einer Vermeidung von Austrocknungen und entzündlichen Irritationen der Nasenschleimhäute.

Zu diesem Zweck wird die erfindungsgemäße pharmazeutische Zusammensetzung im allgemeinen intranasal appliziert, insbesondere mehrmals täglich. Die erfindungsgemäße pharmazeutische Zusammensetzung führt zu einer Abschwellung bzw. Vasokonstriktion der betroffenen Nasenschleimhäute und auf diese Weise zu einer Normalisierung der vermehrten Schleimhautdurchblutung sowie zu einer Reduktion der Ödembildung, einhergehend mit einer Verbesserung der nasalen Ventilation, insbesondere einer verbesserten Belüftung der Nebenhöhlen und der Tuben, und somit zu einer Verhinderung eines Sekretstaus. Gleichzeitig werden Austrocknungen und entzündliche Irritationen der Nasenschleimhäute verhindert.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sie jedoch hierauf zu beschränken.

### Ausführungsbeispiele

### Beispiel 1 (nicht erfindungsgemäß)

Zur Herstellung von 100 g einer klaren wäßrigen Lösung werden in ein 250ml-Becherglas 50 g gereinigtes Wasser vorgelegt. Anschließend werden unter Rühren 0,25 g Hyaluronsäure in Form ihres Natriumsalzes als wäßrige Lösung eingetragen. Der Lösung werden anschließend 0,02 g Benzalkoniumchlorid als Konservierungsstoff zugesetzt und gleichermaßen unter Rühren gelöst. Anschließend werden 0,05 g Oxymetazolin in Form des Hydrochlorids zugesetzt. Das Ganze wird mit weiterem Wasser zum Endgewicht von 100,0 g aufgefüllt und homogen gerührt. Die homogene Lösung wird anschließend, gegebenenfalls nach Filtration über neutrale Cellulosefilter, in Enghalsflaschen aus Braunglas zu 10 oder 20 ml abgefüllt, welche wahlweise mit einer Tropfpipette oder einer Sprühdosierpumpe ausgerüstet sind. Zur Anwendung der Lösung werden mehrmals täglich ein oder mehrere Tropfen bzw. ein oder mehrere Sprühstöße in jedes Nasenloch appliziert.

### Beispiel 2 (erfindungsgemäß)

Beispiel 1 wird wiederholt, jedoch mit der Ausnahme, daß 5 g Wasser durch 5 g Dexpanthenol ersetzt werden.

### Beispiel 3 (nicht erfindungsgemäß)

Beispiel 1 wird wiederholt, jedoch mit der Ausnahme, daß der Hyaluronsäureanteil vollständig durch Wasser ersetzt wird. Es resultiert eine nicht erfindungsgemäße Lösung, die als Wirkstoff lediglich das Oxymetazolinhydrochlorid aufweist.

### Beispiel 4

Zehn Patienten mit akuten Rhinitiden wurden sowohl mit der nicht erfindungsgemäßen Lösung gemäß Beispiel 3 als auch entweder mit der nicht erfindungsgemäßen Lösung gemäß Beispiel 1 oder mit der nichterfindungsgemäßen Lösung gemäß Beispiel 2 behandelt, wobei fünf Patienten mit den Lösungen gemäß den Beispielen 1 und 3 behandelt wurden und die übrigen fünf Patienten mit den Lösungen gemäß den Beispielen 2 und 3. Dabei wurde jeweils eine Nasenseite mit der nicht erfindungsgemäßen, Oxymetazolinhydrochlorid allein enthaltenden Lösung gemäß Beispiel 3 und die andere Nasenseite mit der nicht erfindungsgemäßen Zubereitung gemäß Beispiel 1 oder mit der erfindungsgemäßen Zubereitung gemäß Beispiel 2 behandelt, und die behandelten Patienten wurden in regelmäßigen Abständen mittels Rhinoskopie untersucht.

Bei allen Lösungen trat ein vasokonstriktorischer Effekt, bedingt durch das Oxymetazolin, auf. Auffallend und überraschend ist die Beobachtung, daß die von Oxymetazolin ausgehende Reizwirkung bei Applikation der Lösungen gemäß den Beispielen 1 und 2 nicht auftrat, woraus eine größere Compliance der Patienten resultierte, wohingegen im Fall der Lösung gemäß Beispiel 3 Austrocknungen und entzündliche Irritationen auftraten. Der schleimhautabschwellende Effekt erwies sich nach Behandlung mit den Kombinationen gemäß den Beispielen 1 und 2 als wesentlich deutlicher als erwartet: Der Effekt hielt nach Behandlung mit den Lösungen gemäß Beispiel 1 und 2 länger an, und gleichzeitig wurde eine deutlich bessere Wirkung erzielt. Dabei zeigte sich, daß bei intranasaler Applikation sowohl bei der binären Wirkstoffzusammensetzung gemäß Beispiel 1 als auch bei der ternären Wirkstoffzusammensetzung nach Beispiel 2 gegenüber dem Monopräparat ein deutlich verbesserter Effekt auftrat, was sich dadurch zeigte, daß im Falle der Zusammensetzungen gemäß den Beispielen 1 und 2 keine Austrocknungen der NasenSchleimhäute auftraten, wohingegen bei der Monotherapie mit Oxymetazolin allein Schleimhautaustrocknungen mit einhergehenden entzündlichen Irritationen auftraten.

Die rhinoskopischen Untersuchungen zeigen, daß die Anwendung der ternären erfindungsgemäßen Zusammensetzung gemäß Beispiel 2 gegenüber der binären Mischung gemäß Beispiel 1 einen noch gesteigerten Erfolg bewirkt, da die Rhinitiden bzw. Entzündungen noch schneller abklangen.

Aufgrund der zuvor beschriebenen Ergebnisse wurde die Anwendung der Monosubstanz meist nach wenigen Tagen abgebrochen, um mit den Kombinationen der Beispiele 1 bzw. 2 bei deutlich verbessertem Effekt fortgesetzt zu werden.

Die Ergebnisse der Versuche zeigen einen deutlichen Synergismus der Wirkungen des α-Sympathomimeticums Oxymetazolin einerseits und der Hyaluronsäure andererseits, der durch Zugabe von Pantothenol noch gesteigert werden kann; die erfindungsgemäße pharmazeutische Zusammensetzungen führt bei der Behandlung von akuten Rhinitiden zu einer eindrucksvollen Besserung, welche über das Ausmaß der Einzelwirkstoffe deutlich hinausgeht, was den synergistischen Effekt untermauert.

## Patentansprüche

1. Pharmazeutische Zusammensetzung zur prophylaktischen und/ oder kurativen topischen Behandlung von Rhinitiden, enthaltend in Kombination und jeweils bezogen auf die pharmazeutische Zusammensetzung
a) 0,001 bis 1 Gew.-% mindestens eines zur topischen Anwendung geeigneten Sympathomimeticums mit vasokonstriktorischer und/oder schleimhautabschwellender Wirkung oder dessen physiologisch unbedenkliche Salze;
b) 0,01 bis 5 Gew.-% Hyaluronsäure oder deren physiologisch unbedenkliche Salze; und
c) 0,01 bis 15 Gew.-% Pantothenol oder dessen Ester und/oder Pantothensäure oder deren physiologisch unbedenkliche Salze.

2. Pharmazeutische Zusammensetzung nach Anspruch 1, **dadurch gekennzeichnet, daß** das Sympathomimeticum ein α-Sympathomimeticum, insbesondere ein α-Sympathomimeticum mit 2-Imidazolinstruktur, ist, vorzugsweise aus der Gruppe von Oxymetazolin, Xylometazolin, Tramazolin, Tetryzolin und Naphazolin sowie deren physiologisch unbedenklichen Salzen, besonders bevorzugt Oxymetazolin und/ oder Xylometazolin oder deren physiologisch unbedenkliche Salze.

3. Pharmazeutische Zusammensetzung nach Anspruch 1 oder 2, **dadurch gekennzeichnet, daß** das Sympathomimeticum Oxymetazolinhydrochlorid oder Xylometazolinhydrochlorid ist.

4. Pharmazeutische Zusammensetzung nach mindestens einem der vorangehenden Ansprüche, **dadurch gekennzeichnet, daß** das Sympathomimeticum ausgewählt ist aus der Gruppe von Ephedrin und Ephedrinderivaten, wie Pseudoephedrin, Norephedrin, Norpseudoephedrin, N-Methylephedrin und N-Methylpseudoephedrin.

5. Pharmazeutische Zusammensetzung nach mindestens einem der vorangehenden Ansprüche, **dadurch gekennzeichnet, daß** das Sympathomimeticum in Mengen von 0,01 bis 0,1 Gew.-%, vorzugsweise 0,01 bis 0,05 Gew.-%, bezogen auf die pharmazeutische Zusammensetzung, enthalten ist.

6. Pharmazeutische Zusammensetzung nach mindestens einem der vorangehenden Ansprüche, **dadurch gekennzeichnet, daß** die Hyaluronsäure in Form von deren physiologisch unbedenklichen Salzen, vorzugsweise als Natriumsalz der Hyaluronsäure, eingesetzt ist.

7. Pharmazeutische Zusammensetzung nach mindestens einem der vorangehenden Ansprüche, **dadurch gekennzeichnet, daß** die Hyaluronsäure oder deren physiologisch unbedenkliche Salze in Mengen von 0,05 bis 1 Gew.-%, vorzugsweise 0,05 bis 0,25 Gew.-%, bezogen auf die pharmazeutische Zusammensetzung, enthalten ist.

8. Pharmazeutische Zusammensetzung nach mindestens einem der vorangehenden Ansprüche, **dadurch gekennzeichnet, daß** die pharmazeutische Zusammensetzung Pantothenol oder dessen Ester und/oder Pantothensäure oder deren physiologisch unbedenkliche Salze in Mengen von insgesamt 0,1 bis 10 Gew.-%, vorzugsweise 0,2 bis 5 Gew.-%, bezogen auf die pharmazeutische Zusammensetzung, enthält.

9. Pharmazeutische Zusammensetzung nach einem der Ansprüche 1 bis 8, enthaltend in Kombination und jeweils bezogen auf die pharmazeutische Zusammensetzung
a) 0,001 bis 1 Gew.-%, insbesondere 0,01 bis 0,1 Gew.-%, vorzugsweise 0,01 bis 0,05 Gew.-%, Oxymetazolin und/ oder Xylometazolin, vorzugsweise in Form ihrer physiologisch unbedenklichen Salze, insbesondere in Hydrochloridform;
b) 0,01 bis 5 Gew.-%, insbesondere 0,05 bis 1 Gew.-%, vorzugsweise 0,05 bis 0,25 Gew.-%, Hyaluronsäure, vorzugsweise in Form ihrer physiologisch unbedenklichen Salze, insbesondere als Natriumsalz; und
c) 0,01 bis 15 Gew.-%, insbesondere 0,1 bis 10 Gew.-%, vorzugsweise 0,2 bis 5 Gew.-%, Pantothenol oder dessen Ester und/oder Pantothensäure oder deren physiologisch unbedenkliche Salze.

10. Pharmazeutische Zusammensetzung nach mindestens einem der vorangehenden Ansprüche, **dadurch gekennzeichnet, daß** sie außerdem übliche pharmazeutische Träger-, Zusatz- und/oder Hilfsstoffe enthält.

11. Pharmazeutische Zusammensetzung nach mindestens einem der vorangehenden Ansprüche, **dadurch gekennzeichnet, daß** die Zusammensetzung als Lösung, Dispersion oder Paste, insbesondere als Spray, Salbe, Creme oder Gel, formuliert ist.

12. Verwendung einer pharmazeutischen Zusammensetzung nach einem der Ansprüche 1 bis 11 zur prophylaktischen und/oder kurativen topischen Behandlung von Rhinitiden und/oder zur Herstellung eines Arzneimittels zur prophylaktischen und/oder kurativen topischen Behandlung von Rhinitiden.

13. Verwendung nach Anspruch 12 zur Behandlung von Rhinitiden aller Art, insbesondere Rhinitis acuta, Rhinitis allergica, Rhinitis atrophicans, Rhinitis hyperplastica oder hypertrophicans, Rhinitis mutilans, Rhinitis nervosa oder vasomotorica, Rhinitis pseudomembranacea und Rhinitis sicca.

14. Verwendung nach Anspruch 12 oder 13, **dadurch gekennzeichnet, daß** die pharmazeutische Zusammensetzung nach einem der Ansprüche 1 bis 10 intranasal appliziert wird.

15. Verwendung nach einem der Ansprüche 12 bis 14 zur Abschwellung und/oder Vasokonstriktion der Nasenschleimhäute und/oder zur Normalisierung der vermehrten Schleimhautdurchblutung und/oder zur Reduktion der Ödembildung und/oder zur Verbesserung der nasalen Ventilation, insbesondere zur Belüftung der Nebenhöhlen und der Tuben, und/ oder zur Verhinderung eines Sekretstaus und/oder zur Verhinderung von Austrocknungen und entzündliche Irritationen der Nasenschleimhäute.

## Claims

1. Pharmaceutical composition for the prophylactic and/or curative topical treatment of rhinitis, the pharmaceutical composition comprising in combination and in each case based on the pharmaceutical composition:
a) 0.001 to 1% by weight of at least one sympathomimetic suitable for topical application and having vasoconstrictor action and/or detumescent action on the mucous membrane or its physiologically acceptable salts;
b) 0.01 to 5% by weight of hyaluronic acid or its physiologically acceptable salts; and
c) 0.01 to 15% by weight of pantothenol or its esters and/or pantothenic acid or its physiologically acceptable salts.

2. Pharmaceutical composition according to Claim 1, **characterized in that** the sympathomimetic is an α-sympathomimetic, in particular an α-sympathomimetic having a 2-imidazoline structure, preferably from the group consisting of oxymetazoline, xylometazoline, tramazoline, tetryzoline and naphazoline, and their physiologically acceptable salts, particularly preferably oxymetazoline and/or xylometazoline or their physiologically acceptable salts.

3. Pharmaceutical composition according to Claim 1 or 2, **characterized in that** the sympathomimetic is oxymetazoline hydrochloride or xylometazoline hydrochloride.

4. Pharmaceutical composition according to at least one of the preceding claims, **characterized in that** the sympathomimetic is selected from the group consisting of ephedrine and ephedrine derivatives, such as pseudoephedrine, norephedrine, nor-pseudoephedrine, N-methylephedrine and N-methyl-pseudoephedrine.

5. Pharmaceutical composition according to at least one of the preceding claims, **characterized in that** the sympathomimetic is present in amounts from 0.01 to 0.1% by weight, preferably 0.01 to 0.05% by weight, based on the pharmaceutical composition.

6. Pharmaceutical composition according to at least one of the preceding claims, **characterized in that** the hyaluronic acid is used in the form of its physiologically acceptable salts, preferably as the sodium salt of hyaluronic acid.

7. Pharmaceutical composition according to at least one of the preceding claims, **characterized in that** the hyaluronic acid or its physiologically acceptable salts is present in amounts from 0.05 to 1% by weight, preferably 0.05 to 0.25% by weight, based on the pharmaceutical composition.

8. Pharmaceutical composition according to at least one of the preceding claims, **characterized in that** the pharmaceutical composition contains pantothenol or its esters and/or pantothenic acid or its physiologically acceptable salts in amounts from altogether 0.1 to 10% by weight, preferably 0.2 to 5% by weight, based on the pharmaceutical composition.

9. Pharmaceutical composition according to one of Claims 1 to 8, comprising in combination and in each case based on the pharmaceutical composition
a) 0.001 to 1% by weight, in particular 0.01 to 0.1 % by weight, preferably 0.01 to 0.05% by weight, of oxymetazoline and/or xylometazoline, preferably in the form of their physiologically acceptable salts, in particular in hydro-chloride form;
b) 0.01 to 5% by weight, in particular 0.05 to 1% by weight, preferably 0.05 to 0.25% by weight, of hyaluronic acid, preferably in the form of its physiologically acceptable salts, in particular as the sodium salt; and
c) 0.01 to 15% by weight, in particular 0.1 to 10% by weight, preferably 0.2 to 5% by weight, of pantothenol or its esters and/or pantothenic acid or its physiologically acceptable salts.

10. Pharmaceutical composition according to at least one of the preceding claims, **characterized in that** it moreover contains customary pharmaceutical vehicles, additives and/or excipients.

11. Pharmaceutical composition according to at least one of the preceding claims, **characterized in that** the composition is formulated as a solution, dispersion or paste, in particular as a spray, ointment, cream or gel.

12. Use of a pharmaceutical composition according to one of Claims 1 to 11 for the prophylactic and/or curative topical treatment of rhinitis and/or for the production of a medicament for the prophylactic and/or curative topical treatment of rhinitis.

13. Use according to Claim 12 for the treatment of rhinitis of all types, in particular rhinitis acuta, rhinitis allergica, rhinitis atrophicans, rhinitis hyperplastica or hypertrophicans, rhinitis mutilans, rhinitis nervosa or vasomotorica, rhinitis pseudomembranacea and rhinitis sicca.

14. Use according to Claim 12 or 13, **characterized in that** the pharmaceutical composition according to one of Claims 1 to 10 is administered intranasally.

15. Use according to one of Claims 12 to 14 for the detumescence and/or vasoconstriction of the nasal mucous membranes and/or for the normalization of increased mucous membrane circulation and/or for the reduction of the formation of oedema and/or for the improvement of nasal ventilation, in particular for the ventilation of the nasal sinuses and the eustachian tubes, and/or for the prevention of a secretion blockage and/or for the prevention of drying out and inflammatory irritation of the nasal mucous membranes.

## Revendications

1. Préparation pharmaceutique pour le traitement prophylactique et/ou curatif, topique de rhinites, la préparation pharmaceutique contenant en combinaison et à chaque fois par rapport à la préparation pharmaceutique:
a) 0,001 à 1% en poids d'au moins un sympathomimétique approprié pour une utilisation topique avec un effet vasoconstricteur et/ou de dégonflement des muqueuses ou ses sels physiologiquement compatibles;
b) 0,01 à 5% en poids d'acide hyaluronique ou ses sels physiologiquement compatibles; et
c) 0,01 à 15% en poids de pantothénol ou ses esters et/ou d'acide pantothénique ou ses sels physiologiquement compatibles.

2. Préparation pharmaceutique selon la revendication 1, **caractérisée en ce que** le sympathomimétique est un α-sympathomimétique, en particulier un α-sympathomimétique avec une structure 2-imidazoline, de préférence du groupe formé par l'oxymétazoline, la xylométazoline, la tramazoline, la tétryzoline et la naphtazoline ainsi que leurs sels physiologiquement inoffensifs, de manière particulièrement préférée l'oxymétazoline et/ou la xylométazoline ou leurs sels physiologiquement inoffensifs.

3. Préparation pharmaceutique selon la revendication 1 ou 2, **caractérisée en ce que** le sympathomimétique est le chlorhydrate d'oxymétazoline ou le chlorhydrate de xylométazoline.

4. Préparation pharmaceutique selon au moins l'une quelconque des revendications précédentes, **caractérisée en ce que** le sympathomimétique est choisi dans le groupe formé par l'éphédrine et les dérivés d'éphédrine, tels que la pseudo-éphédrine, la noréphédrine, la norpseudo-éphédrine, la N-méthyléphédrine et la N-méthylpseudo-éphédrine.

5. Préparation pharmaceutique selon au moins l'une quelconque des revendications précédentes, **caractérisée en ce que** le sympathomimétique est contenu en des quantités de 0,01 à 0,1% en poids, de préférence de 0,01 à 0,05% en poids, par rapport à la composition pharmaceutique.

6. Préparation pharmaceutique selon au moins l'une quelconque des revendications précédentes, **caractérisée en ce que** l'acide hyaluronique est utilisé sous forme de ses sels physiologiquement inoffensifs, de préférence sous forme de sel de sodium de l'acide hyaluronique.

7. Préparation pharmaceutique selon au moins l'une quelconque des revendications précédentes, **caractérisée en ce que** l'acide hyaluronique ou ses sels physiologiquement inoffensifs est contenu en des quantités de 0,05 à 1% en poids, de préférence de 0,05 à 0,25% en poids, par rapport à la composition pharmaceutique.

8. Préparation pharmaceutique selon au moins l'une quelconque des revendications précédentes, **caractérisée en ce que** la préparation pharmaceutique contient du pantothénol ou ses esters et/ou l'acide pantothénique ou ses sels physiologiquement inoffensifs en des quantités au total de 0,1 à 10% en poids, de préférence de 0,2 à 5% en poids, par rapport à la préparation pharmaceutique.

9. Préparation pharmaceutique selon l'une quelconque des revendications 1 à 8, contenant en combinaison et à chaque fois par rapport à la préparation pharmaceutique
a) 0,001 à 1% en poids, en particulier 0,01 à 0, 1 % en poids, de préférence 0,01 à 0,05% en poids, d'oxymétazoline et/ou de xylométazoline, de préférence sous forme de leurs sels physiologiquement inoffensifs, en particulier sous forme de chlorhydrate;
b) 0,01 à 5% en poids, en particulier 0,05 à 1% en poids, de préférence 0,05 à 0,25% en poids, d'acide hyaluronique, de préférence sous forme de ses sels physiologiquement inoffensifs, en particulier sous forme de sel sodique; et
c) 0,01 à 15% en poids, en particulier 0,1 à 10% en poids, de préférence 0,2 à 5% en poids, de pantothénol ou ses esters et/ou d'acide pantothénique ou ses sels physiologiquement inoffensifs.

10. Préparation pharmaceutique selon au moins l'une quelconque des revendications précédentes, **caractérisée en ce qu'**elle contient en outre des supports, des additifs et/ou des adjuvants pharmaceutiques usuels.

11. Préparation pharmaceutique selon au moins l'une quelconque des revendications précédentes, **caractérisée en ce que** la préparation est formulée sous forme de solution, de dispersion ou de pâte, en particulier sous forme de spray, de pommade, de crème ou de gel.

12. Utilisation d'une préparation pharmaceutique selon l'une quelconque des revendications 1 à 11 pour le traitement topique prophylactique et/ou curatif de rhinites et/ou pour la préparation d'un médicament destiné au traitement topique prophylactique et/ou curatif de rhinites.

13. Utilisation selon la revendication 12 pour le traitement de rhinites de tous types, en particulier de Rhinitis acuta, de Rhinitis allergica, de Rhinitis atrophicans, de Rhinitis hyperplastica ou hypertrophicans, de Rhinitis mutilans, de Rhinitis nervosa ou vasomotorica, de Rhinitis pseudomembranacea et de Rhinitis sicca.

14. Utilisation selon la revendication 12 ou 13, **caractérisée en ce que** la préparation pharmaceutique selon l'une quelconque des revendications 1 à 10 est appliquée par voie intranasale.

15. Utilisation selon l'une quelconque des revendications 12 à 14 pour le dégonflement et/ou la vasoconstriction des muqueuses nasales et/ou pour la normalisation de l'irrigation augmentée des muqueuses et/ou pour la réduction de la formation d'oedème et/ou pour l'amélioration de la ventilation nasale, en particulier pour aérer les sinus et les conduits et/ou pour éviter un bouchon de sécrétions et/ou pour éviter des dessèchements et des irritations inflammatoires des muqueuses nasales.
